# EUROPEAN PATENT APPLICATION

(11) **EP 2 786 778 A1**
(43) Date of publication of application: **08.10.2014**
(21) Application number: 13162551.9
(22) Date of filing: 05.04.2013
(51) Int. Cl.: A61N 1/05, A61N 1/36, A61N 1/372

(54) **A system for planning and/or providing a therapy for neural applications**

(71) Applicant: Sapiens Steering Brain Stimulation B.V., 5656 AE Eindhoven (NL)
(72) Inventor: Åström, Mattias, 5509 KD Veldhoven (NL); Kleibeuker, Johan Gerard, 5268 HR Helvoirt (NL); Martens, Hubert Cécile François, 5611 ND Eindhoven (NL)
(74) Representative: Prinz & Partner

(57) **Abstract**

The present invention relates to a system (10) for planning and/or providing a therapy for neural applications, especially for neurostimulation and/or neurorecording applications, comprising at least one lead (300), the lead (300) having a plurality of electrodes (132) being capable to provide at least one stimulation field (F), further comprising at least one adjustment means (400) being capable to adjust the stimulation field in multiple directions and at least one steering means (410) being capable to steer the stimulation field in multiple directions simultaneously.

## Description

The present invention relates to a system for planning and/or providing a therapy for neural applications, especially for neurostimulation and/or neurorecording applications.

Implantable neurostimulation devices have been used for the past ten years to treat acute or chronic neurological conditions. Deep brain stimulation (DBS), the mild electrical stimulation of sub-cortical structures, belongs to this category of implantable devices, and has been shown to be therapeutically effective for Parkinson's disease, Dystonia, and Tremor. New applications of DBS in the domain of psychiatric disorders (obsessive compulsive disorder, depression) are being researched and show promising results. In existing systems, the probes are connected to an implantable current pulse generator.

Currently, systems are under development with more, smaller electrodes in a technology based on thin film manufacturing. These novel systems consist of a lead made from a thin film based on thin film technology, as e.g. described in WO 2010/055453 A1. The thin film leads are fixed on a carrier material to form a probe. These probes will have multiple electrode areas and will enhance the precision to address the appropriate target in the brain and relax the specification of positioning. Meanwhile, undesired side effects due to undesired stimulation of neighbouring areas can be minimized.

Leads that are based on thin film manufacturing are e.g. described by US 7,941,202 and have been used in research products in animal studies.

In existing systems, the DBS lead has e.g. four 1.5 mm-wide cylindrical electrodes at the distal end spaced by 0.5 mm or 1.5 mm. The diameter of the lead is 1.27 mm and the metal used for the electrodes and the interconnect wires is an alloy of platinum and iridium. The coiled interconnect wires are insulated individually by fluoropolymer coating and protected in an 80A urethane tubing. With such electrode design, the current distribution emanates uniformly around the circumference of the electrode, which leads to stimulation of all areas surrounding the electrode.

The lack of fine spatial control over field distributions implies that stimulation easily spreads into adjacent structures inducing adverse side-effects in about 30% of the patients. To overcome this problem, systems with high density electrode arrays are being developed, hence providing the ability to steer the stimulation field to the appropriate target.

Deep brain stimulation (DBS) is an effective treatment for movement disorders such as Parkinson's disease. DBS leads are implanted with stereotactic neurosurgical techniques in the deep regions of the brain. Chronic electrical stimulation is delivered to the electrodes from battery-operated pulse generators that are implanted below the clavicle. The clinical benefit of DBS is largely dependent on the spatial distribution of the stimulation field in relation to brain anatomy. To maximize therapeutic benefits while avoiding unwanted side-effects, precise control over the stimulation field is essential.

Currently, the programming procedure of neurostimulation devices is performed by defining the active electrode contacts, the amplitudes (current or electric potential) of one or more electrical sources that are applied to the active electrode contacts, as well as a few other electrical parameters that define the electrical pulses that are delivered to the patient. The patient is assessed and the electrical settings are adjusted according to certain programming algorithms in order to reach an optimal therapeutic outcome. The procedure is performed without any visual aid of the distribution of the stimulation field.

Novel DBS devices are currently being developed that allow asymmetrical steering of the stimulation field in relation to the principal axes of the stimulation lead. The ability to steer the stimulation field is from now on denoted as steering brain stimulation. Steering brain stimulation provides additional possibilities as well as complexity to the programming procedure. In order to keep the programming procedure reasonable quick and to make use of the added features there is a need for tools that support the programming procedure. One way to support the programming procedure is to visualize the distribution of the stimulation field and provide tools for the programmer to interact and control the distribution of the stimulation field.

During programming of neurostimulator devices with steering brain stimulation capabilities, the programmer may be faced with a large number of electrode contacts (n > 10) to which electrical settings can be applied. When the electric field is asymmetrically steered in one (or several) directions the electrical settings of individual electrode contacts need to be adjusted. In order to keep the programming procedure reasonable time-efficient it is not feasible to manually adjust the electrical settings on an individual contact basis. Thus, there is a need for a tool with intuitive controls with which the programmer can steer the stimulation field.

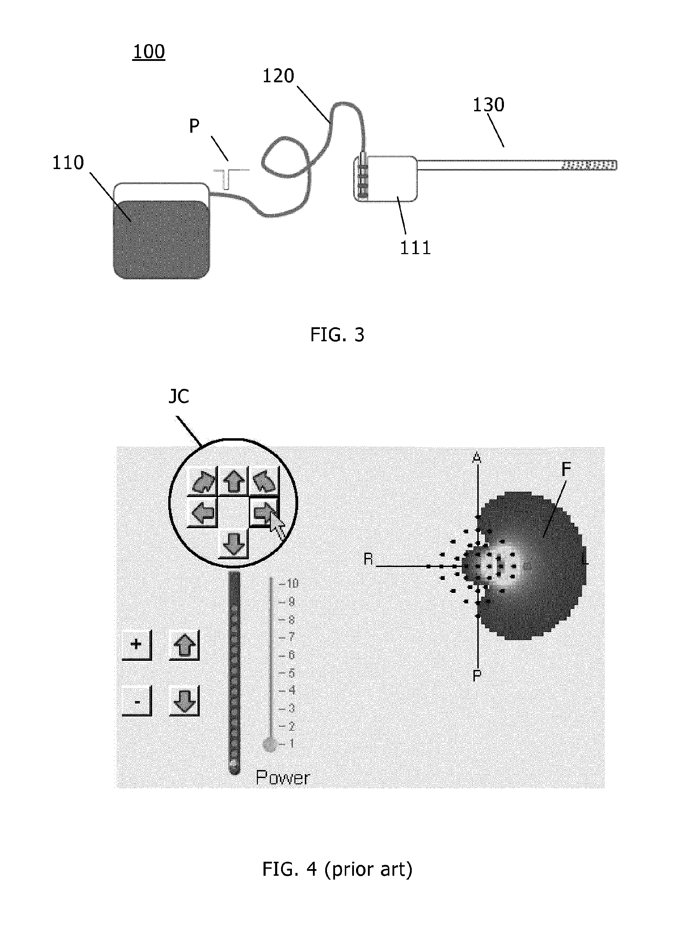

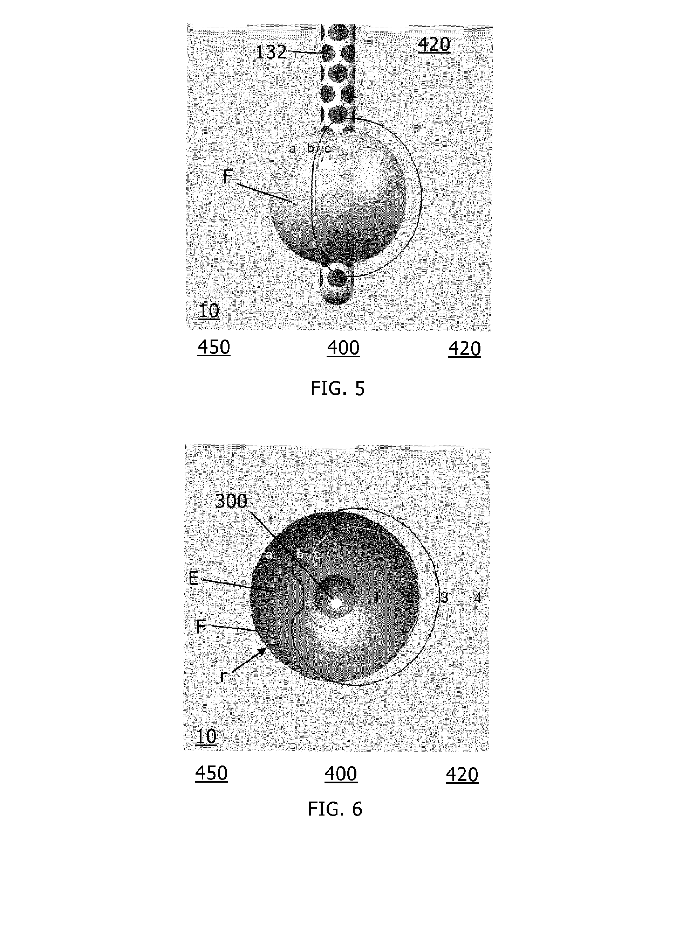

Stimulation fields from devices that provide steering functionality are commonly controlled with buttons that are organized in a joystick like manner (see e.g. Figure 4). With a joystick control the stimulation field is moved asymmetrically around the centre of the lead. However, moving the field into a certain direction may from a clinical point of view not be the most intuitive way of controlling the field during neurostimulation. It may be hypothesised that steering is primarily going to be used when unwanted stimulation-induced side-effects are encountered. In such case it is hypothesised that the user would like to turn off or reduce intensity of certain parts of the field in the anatomical direction where the side effect is suspected to be related, rather than moving the field away from a certain direction where the field will enter new tissue in the opposite direction (Figure 5 and 6). Another limitation of the joystick approach is that the field can only be steered in one direction at a time. Thus, there is a need for a steering means that can provide intuitive control of the stimulation field during steering without limiting the degrees of freedom provided by the stimulation hardware device.

It is therefore an object of the present invention to improve a system for planning and/or providing a therapy for neural applications, especially for neurostimulation and/or neurorecording applications, in particular in that a tool with intuitive controls with which the programmer can steer the stimulation field and a steering means that can provide intuitive control of the stimulation field during steering without limiting the degrees of freedom provided by the stimulation hardware device is provided.

The above object is solved with a system for planning and/or providing a therapy for neural applications with the features of claim 1. Accordingly, a system for planning and/or providing a therapy for neural applications is provided, especially for neurostimulation and/or neurorecording applications, comprising at least one lead, the lead having a plurality of electrodes being capable to provide at least one stimulation field, further comprising at least one adjustment means being capable to adjust the stimulation field in multiple directions and at least one steering means being capable to steer the stimulation field in multiple directions simultaneously.

By this, the advantage is achieved that a tool with intuitive controls is provided with which the programmer can steer the stimulation field and a steering means that can provide intuitive control of the stimulation field during steering without limiting the degrees of freedom provided by the stimulation hardware device.

Especially, the lead may be a lead for neural applications, preferably a lead for a neurostimulation and/or neurorecording system. Such a neurostimulation and/or neurorecording system may be e.g. a DBS system.

The lead may e.g. comprise at least one thin film, whereby the thin film comprises a proximal end and a distal end, the lead further comprising a plurality of electrodes on the distal end of the thin film.

The thin film may include at least one electrically conductive layer, preferably made of a biocompatible material. The thin film may be assembled to the carrier and further processed to constitute the lead element. The thin film for a lead is preferably formed by a thin film product having a distal end, a cable with metal tracks and a proximal end. The distal end of the thin film may be forming a part of the distal end of the lead or substantially the distal end of the lead.

The distal end of the lead may be the end of the lead, which is in the implanted state of the lead the remote end of the lead with regard to the body surface area. In particular, in case of a lead for brain application, the distal end of the lead is the lower end of the lead, which is remote to the burr-hole of the skull, through which the lead is implanted.

There may be an Advanced Lead Can element, which may comprise electronic means to address the plurality of electrodes and at least one Advanced Lead Can connecting means. Further, the Advanced Lead Can element may be hermetically or substantially hermetically sealed and may comprise electronic means to address the plurality of electrodes on the distal end of the thin film, which is arranged at the distal end and next to the distal tip of the lead. The plurality of electrodes may comprise more than 5-10 electrodes, e.g. 16 or 32 electrodes or in preferred embodiments e.g. 64 electrodes or more. The electrodes may be arranged such that the electrodes are substantially evenly distributed arranged all over the distal end of the lead.

For instance, it is advantageously possible that the adjustment means is configured such that the shape of the at least one stimulation field can be modelled such that user input via the steering means is transformed into the shape of the stimulation field. By this, the advantage is achieved that no adjustment of the parameters of each and every electrode must be input by the user. It is possible to form the desired stimulation field directly and so the parameters of the electrodes involved to provide the required and desired shape of the stimulation field are correctly set, advantageously automatically by the system.

The adjustment means may comprise one or more controllers and/or processing means and/or the necessary calculation means and/or storing means and/or input means and/or output means to be capable that the adjustment means is configured such that the shape of the at least one stimulation field can be modelled such that user input via the steering means is translated into the shape of the stimulation field.

Furthermore, it is possible that the adjustment means is configured such that the shape of the stimulation field is transformed into stimulation settings to provide the at least one stimulation field with electrodes of the lead.

Advantageously, it is possible that the adjustment means is configured such that the stimulation field is automatically directly and/or indirectly adjusted.

Furthermore, it is possible that the plurality of electrodes forms a complex geometrical array and/or that the plurality of electrodes is arranged circumferentially around at least one section of the lead, especially around a section next to the distal tip end of the lead.

A complex array may be formed by a plurality of electrodes, which are arranged circumferentially around a section next to the distal tip end of the lead. The electrodes may be e.g. arranged non-planar and non-coaxial and likewise a leopard pattern and may form a regular array. Several electrodes may form at one level a ring around the lead and the next ring may be slightly displaced such that e.g. one electrode of the second ring is partially arranged within the gap between to electrodes of the first ring. So, there are rings of electrodes in radial/circumferential direction of the lead and columns of electrodes in axial direction of the lead.

Moreover, it is possible that the adjustment means comprises at least one the visualization means which is configured such that the lead and the at least one stimulation field can be visualized, wherein the steering means and the visualization means are interconnected such that a geometrical interrelation of input and visualization is provided.

By this the advantage is achieved that the at least one characteristic parameter of the stimulation field can be intuitively input by the user, since due to the geometrical interrelation of input and visualization it is clear at which location of the stimulation field an amend of the at least one characteristic parameter of the stimulation field will cause an amendment of the stimulation field. Advantageously, by means of the interconnection input means and visualization means and the geometrical interrelation of input and visualization, the effect of the change is immediately visualized in a "what-you-see-is-what-you-get-manner" and thus creating an intuitive input possibility combined with a fast and accurate adjustment of the stimulation field.

It is further possible that the visualization means is configured such that the at least one stimulation field is displayed around an axial top view of the lead (300).

So, beyond the constantly updated visualization of the stimulation field the user shall be enabled to interpret the status of the stimulation field immediately.

Moreover, it is possible that the steering means comprises one or more first input points radially arranged around the visualization of the lead, wherein especially the first input points are capable to adjust the at least one stimulation field in radial direction.

In respect of a simplified visualization of the stimulation field by an axial top view, the input means may be embodied as one or more input points radially arranged around the visualization of the lead. So, the interconnection between input means and the visualization means is visualized and provides the possibility of an instant adjustment of the stimulation field.

It is further possible that the steering means comprises one or more second input points arranged adjacent, especially parallel, to the visualization of the lead, wherein especially the second input points are capable to adjust the at least one stimulation field in axial direction.

By this the advantage is achieved that e.g. the relevant electrodes along the axis of the lead may be switched on and/or off and may be e.g. adjusted regarding their output. In case that the arrangement is parallel it is immediately clear at which level the electrodes are activated and adjusted. Further, it is possible that for example controls e.g. of the the relevant electrodes along the axis of the lead may be provided which may result in specific configurations of stimulation settings.

Additionally, it is possible that the first input points and/or second input points of the steering means are configured such that the first input points and/or second input points can be switched between two or more states related to at least one stimulation field characteristic.

The states may be e.g. 'On', 'Off', 'Half', 'Quarter', or other values related to the stimulation amplitude or the like.

Additionally, it is possible that the at least one characteristic of the stimulation field is the stimulation amplitude and/or stimulation energy and/or the pulse-width and/or parameters which are directly and/or indirectly influencing the stimulation amplitude and/or stimulation energy and/or the pulse-width.

Moreover, it is possible that the first input points and/or second input points of the steering means are configured such that the first input points and/or second input points can be arranged in a first rotated arrangement to an unfolded visualization of at least a part of electrodes provided by the visualization means and that the first input points and/or second input points can be arranged in a second aligned arrangement to an unfolded visualization of at least a part of electrodes provided by the visualization means.

The part of electrodes may be a ring of electrodes, especially two adjacent rings of electrodes. The first rotated arrangement to an unfolded visualization of at least one part of electrodes may be a visualization setup, wherein the electrodes of the ring next to the input points are arranged such that two input points are associated to at least one electrode. The second aligned arrangement to an unfolded visualization of at least one part of electrodes may be a visualization setup, wherein the electrodes of the ring next to the input points are arranged such that one input point is associated to the electrode, the next input point is associated to a gap between two adjacent electrodes etc.

It is possible that it may be switched between the first rotated arrangement to the second aligned arrangement and vice versa.

Additionally, it is possible that the steering means is configured such that the stimulation field may be at least partially tilted and/or sloped and/or rotated. Tilting and/or sloping and/or rotating may relate to shifting the active electrode contacts on one or more sides of the stimulation lead in the vertical direction. The effect is that the original field is tilted and/or sloped and/or rotated in one or more directions. This feature may become valuable e.g. when the user would like to tailor the stimulation field to cover the motor part of the subthalamic nucleus (STN).

Furthermore, it is possible that the geometrical interrelation of input and visualization is provided such that the visualization of the lead and the visualization of at least one stimulation field is arranged in the center of at least one part of the steering means, especially in the center of the circle of the first input points, wherein further especially the longitudinal axis of the lead being displayed in an axial top view is in the center of the circle of input points and the stimulation field is displayed on an isosurface level which is identical to the level defined by the first input points.

It is further possible that the adjustment means comprises at least one touch screen, wherein the touch screen is configured such that the at least one steering means and the at least one visualization means are provided by the touch screen.

Furthermore, the following method for planning and/or providing a therapy for neural applications, especially a neurostimulation and/or neurorecording applications is disclosed.

Accordingly, the method for planning and/or providing a therapy for neural applications, especially a neurostimulation and/or neurorecording applications comprises at least the step of simulating a stimulation field for a unit amplitude(s) applied to a specific set of active electrode contacts defined by the user.

Further, it is possible that the maximum electric field strength, E, that should be distributed at a radial distance r is defined by the user.

Moreover, it is possible that the maximum electric field strength is measured at the radial distance r in the finite element simulation.

Additionally, it is possible that the ratio between the measured field strength and the desired field strength is calculated.

Furthermore, the unit amplitude(s) that was used during the simulation is multiplied with this ratio. The result is the amplitude required to produce the desired field.

These steps may be combined and preferably all mentioned steps of the method for planning and/or providing a therapy for neural applications, especially a neurostimulation and/or neurorecording applications are carried out in the following order:
1. A stimulation field is simulated for a unit amplitude(s) applied to a specific set of active electrode contacts defined by the user.
2. The maximum electric field strength, E, that should be distributed at a radial distance, r, are both defined by the user.
3. The maximum electric field strength is measured at the radial distance r in the finite element simulation.
4. The ratio between the measured field strength and the desired field strength is calculated.
5. The unit amplitude(s) that was used during the simulation is multiplied with this ratio. The result is the amplitude required to produce the desired field.

In order to display the desired field without having to iterate the simulation of the field using the calculated amplitude, it is possible to display the field with an isosurface level corresponding to the measured field strength in the tissue. This can be done since the size and shape of that isosurface will be identical to the desired isosurface after recalculating the field with the calculated amplitude.

Radial controlled stimulation can also be used to keep a constant but unspecified maximum field radius when the active electrode contact configuration is changed. In such case the radial distance r of the stimulation field is measured in the finite element simulation generated by the previous stimulation settings. This way the stimulation field radius is kept constant to the previous stimulation field settings.

Further details and advantages of the present invention shall be described hereinafter with respect to the drawings:
- Fig. 1:: a schematical drawing of a neurostimulation system for deep brain stimulation (DBS);
- Fig. 2:: a further schematical drawing of a probe of a neurostimulation system for deep brain stimulation (DBS) and its components;
- Fig. 3:: a schematical drawing of a probe system according to the present invention;
- Fig. 4:: a steering system according to the prior art;
- Fig. 5:: a side (anterior) view of a simulated electrical field around a lead;
- Fig. 6:: a top (superior) view of a simulated electrical field around a lead;
- Fig. 7:: a top (superior) view of a simulated electrical field around a lead provided by a system according to an embodiment of the present invention;
- Fig. 8:: a side view of the distal end of the lead with the complex array of electrodes;
- Fig. 9:: a rotated graphical user interface and an aligned graphical user interface of the system according to an embodiment of the present invention;
- Fig. 10:: a view of the graphical user interface of the system according to an embodiment of the present invention;
- Fig. 11:: a further view of the graphical user interface of the system according to an embodiment of the present invention;
- Fig. 12:: a further view of the graphical user interface of the system according to an embodiment of the present invention;
- Fig. 13:: a first view of the graphical user interface of the system according to an embodiment of the present invention during tilting; and
- Fig. 14:: a second view of the graphical user interface of the system according to an embodiment of the present invention during tilting.

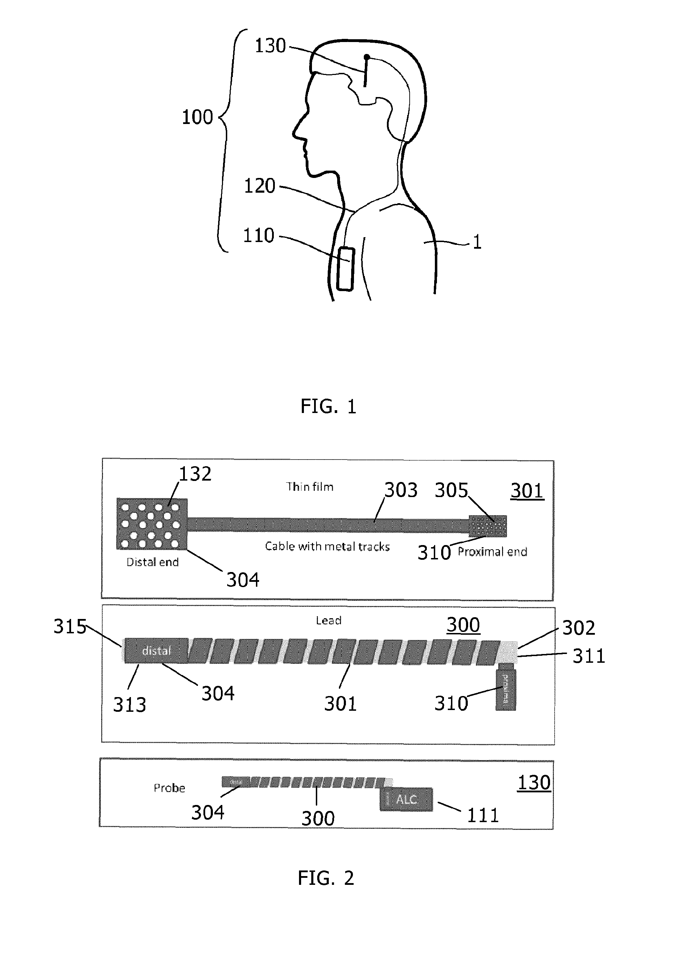

A possible embodiment of a neurostimulation system 100 for deep brain stimulation (DBS) is shown in Figure 1. The neurostimulation system 100 comprises at least a controller 110 that may be surgically implanted in the chest region of a patient 1, typically below the clavicle or in the abdominal region of a patient 1. The controller 110 can be adapted to supply the necessary voltage pulses. The typical DBS system 100 may further include an extension wire 120 connected to the controller 110 and running subcutaneously to the skull, preferably along the neck, where it terminates in a connector. A DBS lead arrangement 130 may be implanted in the brain tissue, e.g. through a burr-hole in the skull.

Figure 2 further illustrates a typical architecture for a Deep Brain Stimulation probe 130 that comprises a DBS lead 300 and an Advanced Lead Can element 111 comprising electronic means to address electrodes 132 on the distal end 304 of the thin film 301, which is arranged at the distal end 313 and next to the distal tip 315 of the DBS lead 300. The lead 300 comprises a carrier 302 for a thin film 301, said carrier 302 providing the mechanical configuration of the DBS lead 300 and the thin film 301. The thin film 301 may include at least one electrically conductive layer, preferably made of a biocompatible material. The thin film 301 is assembled to the carrier 302 and further processed to constitute the lead element 300. The thin film 301 for a lead is preferably formed by a thin film product having a distal end 304, a cable 303 with metal tracks and a proximal end 310. The proximal end 310 of the thin film 301 arranged at the proximal end 311 of the lead 300 is electrically connected to the Advanced Lead Can element 111. The Advanced Lead Can element 111 comprises the switch matrix of the DBS steering electronics. The distal end 304 comprises the electrodes 132 for the brain stimulation. The proximal end 310 comprises the interconnect contacts 305 for each metal line in the cable 303. The cable 303 comprises metal lines (not shown) to connect each distal electrodes 132 to a designated proximal contact 305.

Figure 3 shows schematically and in greater detail an embodiment of a system 100 for brain applications, here for neurostimulation and/or neurorecording as a deep brain stimulation system 100 as shown in Figures 1 and 2. The probe system 100 comprises at least one probe 130 for brain applications with stimulation and/or recording electrodes 132, whereby e.g. 64 electrodes 132 can be provided on outer body surface at the distal end of the probe 130. By means of the extension wire 120 pulses P supplied by controller 110 can be transmitted to the Advanced Lead Can 111. The controller 110 can be an implantable pulse generator (IPG) 110.

Figure 4 shows steering system according to the prior art. This known system provides a steering functionality are commonly controlled with buttons that are organized in a joystick like manner. With a joystick control JC the stimulation field F is moved asymmetrically around the centre of the lead. However, moving the field F into a certain direction may from a clinical point of view not be the most intuitive way of controlling the field F during neurostimulation. It may be hypothesised that steering is primarily going to be used when unwanted stimulation-induced side-effects are encountered. In such case it is hypothesised that the user would like to turn off certain parts of the field F in the anatomical direction where the side effect is suspected to be related, rather than moving the field away from a certain direction where the field will enter new tissue in the opposite direction. Another limitation of the joystick approach is that the field F can only be steered in one direction at a time.

Figures 5 and 6 show a side (anterior; Figure 5) and top (superior; Figure 6) view of simulated electric fields F with around the lead 300 during a) no steering, b) steering by moving the field with joystick controls, and c) steering by turning off certain parts of the field. The electric fields F may be visualized with coloured (e.g. green) isolevels in 3D, and black and grey contours in 2D at a threshold of e.g. 400 V/m.

The electric field strength E of the Field F may be measured at the radial distance r.

In a possible embodiment of the present invention a system 10 may be a system 10 for planning and/or providing a therapy for neural applications, especially for neurostimulation and/or neurorecording applications, here a system 10 for planning and/or providing a deep brain stimulation (DBS) therapy. This system 10 may realize a graphical user interface concept for neurostimulator devices that allows steering of the stimulation field in multiple directions simultaneously.

In particular, the system 10 comprises at least one lead 300. The lead 300 has a plurality of electrodes 132 being capable to provide at least one stimulation field F. The plurality of electrodes 132 forms a complex geometrical array and/or that the plurality of electrodes 132 is arranged circumferentially around at least one section of the lead, especially around a section next to the distal tip end of the lead 300.

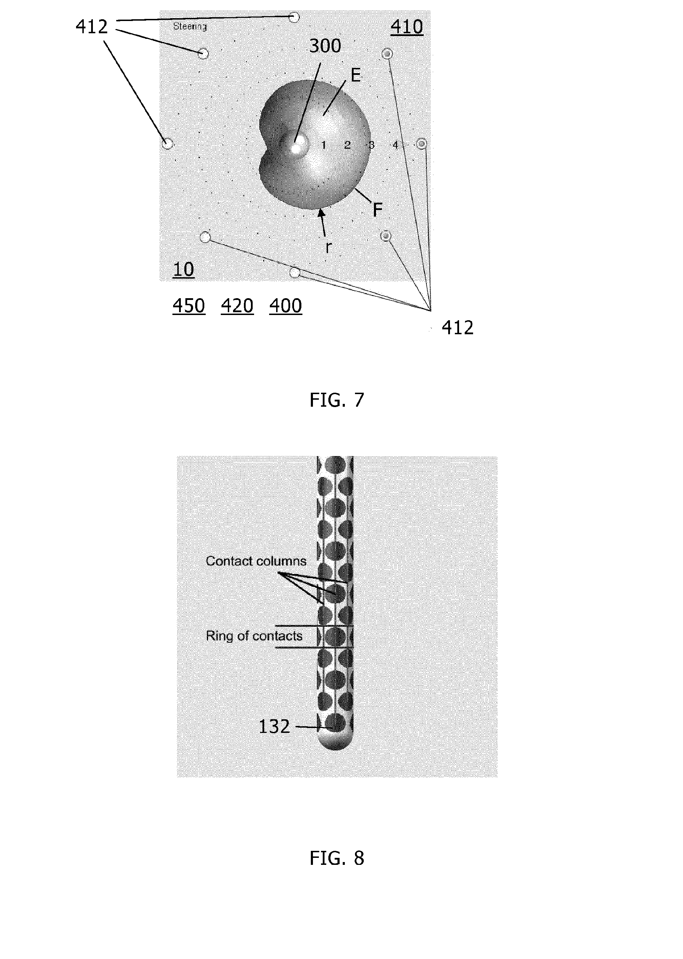

The complex array is formed by a plurality of electrodes 132, which are arranged circumferentially around a section next to the distal tip end of the lead 300 as shown in Figure 8. The electrodes may be e.g. arranged non-planar and non-coaxial and likewise a leopard pattern and may form a regular array. Several electrodes may form at one level a ring around the lead and the next ring may be slightly displaced such that e.g. one electrode of the second ring is partially arranged within the gap between to electrodes of the first ring. So, there are rings of electrodes in radial/circumferential direction of the lead and columns of electrodes in axial direction of the lead.

Further, the system 10 comprises an adjustment means 400 being capable to adjust the stimulation field in multiple directions and a steering means 410 being capable to steer the stimulation field in multiple directions simultaneously. The adjustment means 400 is configured such that the shape of the at least one stimulation field F can be modelled such that user input via the steering means 410 is transformed into the shape of the stimulation field F. Further, the adjustment means 400 is configured such that the shape of the stimulation field F can be transformed into stimulation settings to provide the at least one stimulation field F with the electrodes 132 of the lead 300. Also, the adjustment means 400 is configured such that the stimulation field F is automatically directly and/or indirectly adjusted.

The adjustment means 400 has a touch screen 450, wherein the touch screen 450 is configured such that the steering means 410 and the visualization means 420 are provided by the touch screen 450.

The visualization means 420 is configured such that the lead 300 and the at least one stimulation field F can be visualized, wherein the steering means 410 and the visualization means 420 are interconnected such that a geometrical interrelation of input and visualization is provided. It is possible that the lead 300 is represented with or without an electrode array representation. By not displaying the electrodes of the lead the user may be not focussing on electrodes but more on field shaping which may help to achieve better planning and therapy results. There may be a switching means which is capable to switch between a representation of the lead with or without an electrode array representation.

As can be seen in Figure 7, the visualization means 420 is configured such that the at least one stimulation field is displayed around an axial top view of the lead 300.

The steering means 410 comprises first input points 412 radially arranged around the visualization of the lead 300, wherein especially the first input points 412 are capable to adjust the at least one stimulation field F in radial direction.

So, an intuitive graphical user interface concept for multi-directional steering can be realized, which comprises input points 412 control buttons that are located in the vicinity of a stimulation lead 300.

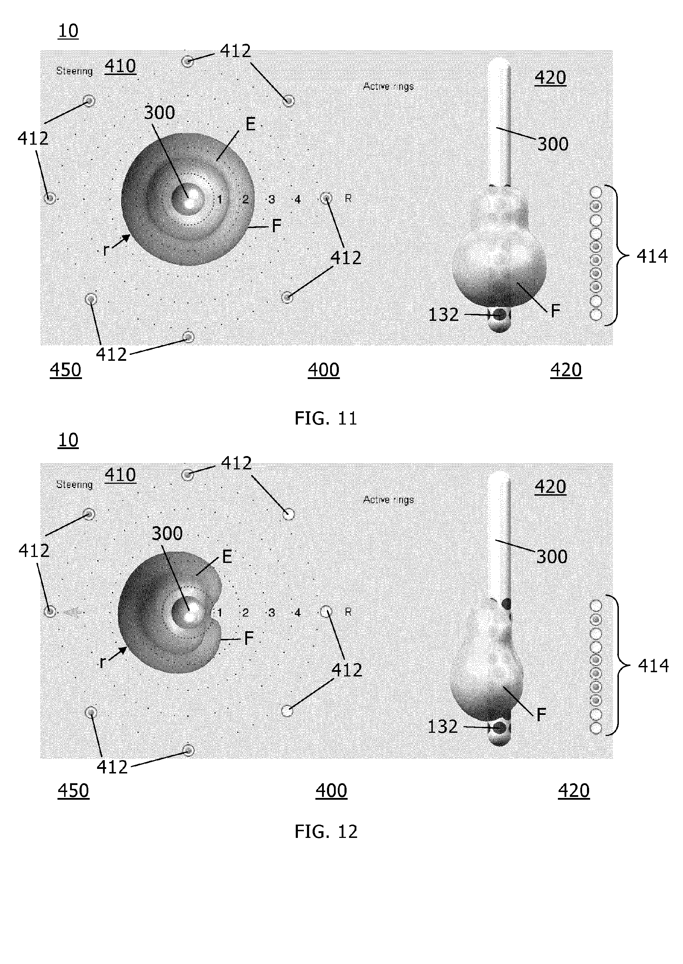

The steering means 410 comprises one or more second input points 414 arranged adjacent and in this case parallel (see e.g. Figures 11 and 12) to the visualization of the lead 300, wherein especially the second input points 414 are capable to adjust the at least one stimulation field F in axial direction.

The first input points 412 or input buttons 412 and the second input points 414 or input buttons 414 of the steering means 410 are configured such that the first input points 412 and second input points 414 can be switched between two or more states related to at least one stimulation field characteristic.

The first input points 412 and also the second input points 414 of the steering means 410 are configured such that the first input points 412 and/or second input points 414 can be switched between two or more states related to at least one stimulation field characteristic.

The at least one stimulation field characteristic is the stimulation amplitude and/or stimulation energy and/or the pulse-width and/or parameters which are directly and/or indirectly influencing the stimulation amplitude and/or stimulation energy and/or the pulse-width.

Each button controls and influences the electrical settings of one or more lead contact columns, see Figure 7. The number of control buttons may not necessarily be the same as the number of lead contact columns. Each control button can have two or more states related to the amount of electrical stimulation amplitude delivered at a certain lead contact column. Such states can be e.g. 'On', 'Off', 'Half', 'Quarter', or other values related to the stimulation amplitude (Figure 4). Radial steering can be applied individually at different vertical levels by a control that states at what vertical level the steering should be applied.

Coloured buttons 412, 414 with a first colour, e.g. green buttons, refer to electrode contact columns or parts of electrode contact columns that are 'On', i.e. to electrode contacts where electric current is delivered, and coloured buttons 412, 414 with a second colour, e.g. white buttons, refer to electrode contact columns or parts of electrode contact columns that are 'Off'. There is not a direct mapping of electrode columns. In particular, if on the staggered array one ore more (but not all) button(s) are activated this may imply that a column is put 'Half-On'. By means of the buttons it may be controlled to which extent the filed in 'On' in the particular direction. The system is configured such that the location on the field is translated into a mapping of electrical energy and/or current transmitted to electrode contacts.

As can be seen in Figures 7 and 10 to 14, the geometrical interrelation of input and visualization is provided such that the visualization of the lead 300 and the visualization of at least one stimulation field F is arranged in the center of at least one part of the steering means 410, especially in the center of the circle of the first input points 412, wherein further especially the longitudinal axis of the lead 300 being displayed in an axial top view is in the center of the circle of input points 412 and the stimulation field is displayed on an isosurface level which is identical to the level defined by the first input points 412.

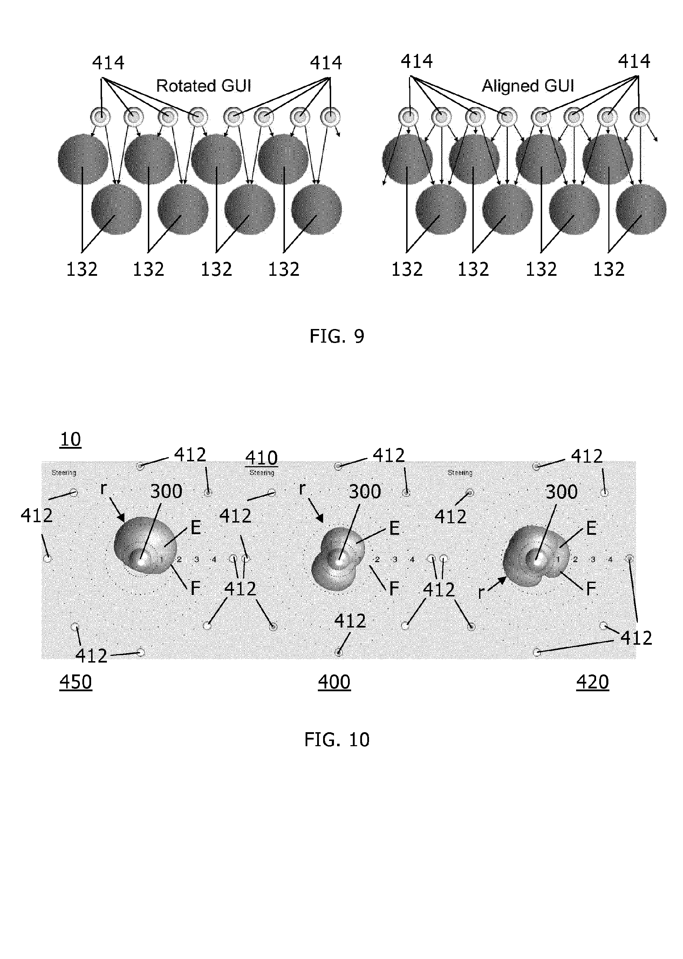

For example the second input points 414 of the steering means 410 are arranged in a first rotated arrangement to an unfolded visualization of at least one part of electrodes 132 provided by the visualization means 420 (see Figure 9, left view) and that the first input points 412 and/or second input points 414 can be arranged in a second aligned arrangement to an unfolded visualization of at least one part of electrodes 132 provided by the visualization means 420 (see Figure 9, right view). For example, by means of the input points 414 the stimulation amplitudes of aligned and/or adjacent contacts according to specific algorithms can be affected and adjusted.

The graphical user interface of the system 10 for multi-directional steering as shown in Figure 10 is used to steer the stimulation field by setting the state of each control button 412 (and likewise also the buttons 414, see e.g. Figures 11 and 12). The number of directions in which the field can be steered simultaneously is primarily related to the number of lead contact columns, but ultimately also to the stimulation amplitudes as well as the size of the lead diameter and in multiple directions at the same time.

As shown in Figures 11 and 12, control buttons 414 are also located in the vicinity of the stimulation lead in the vertical plane. Such control buttons 414 influence the electrical settings of one or more lead contact rings. The number of control buttons 414 may not necessarily be the same as the number of lead contact rings. Each control button can have two or more states related to the amount of electrical stimulation amplitude delivered at a certain lead contact column. Such states can be e.g. 'On', 'Off', 'Half', 'Quarter', or other values related to the stimulation amplitude.

Radial and vertical steering may be combined to generate arbitrary stimulation field shapes of the stimulation field F.

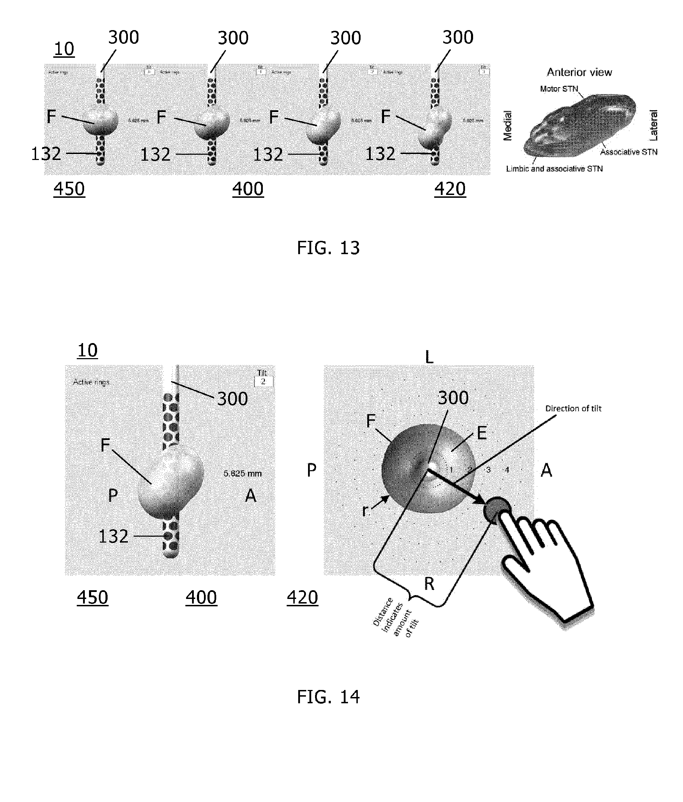

As shown in Figure 13 and 14, the steering means 410 is configured such that the stimulation field F may be at least partially tilted and/or sloped and/or rotated.

In addition to radial and vertical steering there is also control for steering the field F by means of tilt. Tilt refers to shifting the active electrode contacts on one or more sides of the stimulation lead in the vertical direction. The effect is that the original field is tilted in one or more directions. This feature may become valuable e.g. when the programmer would like to tailor the stimulation field to cover the motor part of the subthalamic nucleus (STN).

Figure 14 shows tilted stimulation fields in anterior view (left view), and superior view (right side). An example of a user interface (UI) control for tilt was in this example put in the superior viewport. The direction of tilt is illustrated by the arrow, and the magnitude of the tilt is defined by the radial extension of the arrow.

## Claims

1. A system (10) for planning and/or providing a therapy for neural applications, especially for neurostimulation and/or neurorecording applications, comprising at least one lead (300), the lead (300) having a plurality of electrodes (132) being capable to provide at least one stimulation field (F), further comprising at least one adjustment means (400) being capable to adjust the stimulation field in multiple directions and at least one steering means (410) being capable to steer the stimulation field in multiple directions simultaneously.

2. The system (10) according to claim 1,
**characterized in that**
the adjustment means (400) is configured such that the shape of the at least one stimulation field (F) can be modelled such that user input via the steering means (410) is transformed into the shape of the stimulation field (F).

3. The system (10) according to claim 2,
**characterized in that**
the adjustment means (400) is configured such that the shape of the stimulation field (F) can be transformed into stimulation settings to provide the at least one stimulation field (F) with the electrodes (132) of the lead (300).

4. The system (10) according to one of the preceding claims,
**characterized in that**
the adjustment means (400) is configured such that the stimulation field (F) is automatically directly and/or indirectly adjusted.

5. The system (10) according to one of the preceding claims,
**characterized in that**
the plurality of electrodes (132) forms a complex geometrical array and/or that the plurality of electrodes (132) is arranged circumferentially around at least one section of the lead, especially around a section next to the distal tip end of the lead (300).

6. The system (10) according to one of the preceding claims,
**characterized in that**
the adjustment means (400) comprises at least one visualization means (420) which is configured such that the lead and the at least one stimulation field (F) can be visualized, wherein the steering means (410) and the visualization means (420) are interconnected such that a geometrical interrelation of input and visualization is provided.

7. The system (10) according to claim 6,
**characterized in that**
the visualization means (420) is configured such that the at least one stimulation field is displayed around an axial top view of the lead (300).

8. The system (10) according to one of the preceding claims,
**characterized in that**
the steering means (410) comprises one or more first input points (412) radially arranged around the visualization of the lead (300), wherein especially the first input points (412) are capable to adjust the at least one stimulation field (F) in radial direction.

9. The system (10) according to one of the preceding claims,
**characterized in that**
the steering means (410) comprises one or more second input points (414) arranged adjacent, especially parallel, to the visualization of the lead (300), wherein especially the second input points (414) are capable to adjust the at least one stimulation field (F) in axial direction.

10. The system (10) according to one of claims 8 or 9,
**characterized in that**
the first input points (412) and/or second input points (414) of the steering means (410) are configured such that the first input points (412) and/or second input points (414) can be switched between two or more states related to at least one stimulation field characteristic.

11. The system (10) according to claim 10,
**characterized in that**
the at least one stimulation field characteristic is the stimulation amplitude and/or stimulation energy and/or the pulse-width and/or parameters which are directly and/or indirectly influencing the stimulation amplitude and/or stimulation energy and/or the pulse-width.

12. The system (10) according to one of claims 8 to 11,
**characterized in that**
the first input points (412) and/or second input points (414) of the steering means (410) are configured such that the first input points (412) and/or second input points (414) can be arranged in a first rotated arrangement to an unfolded visualization of at least a part of electrodes (132) provided by the visualization means (420) and that the first input points (412) and/or second input points (414) can be arranged in a second aligned arrangement to an unfolded visualization of at least a part of electrodes (132) provided by the visualization means (420).

13. The system (10) according to one of the preceding claims,
**characterized in that**
the steering means (410) is configured such that the stimulation field (F) may be at least partially tilted and/or sloped and/or rotated.

14. The system (10) according to one of claims 6 to 13,
**characterized in that**
the geometrical interrelation of input and visualization is provided such that the visualization of the lead (300) and the visualization of at least one stimulation field (F) is arranged in the center of at least a part of the steering means (410), especially in the center of the circle of the first input points (412), wherein further especially the longitudinal axial of the lead (300) being displayed in an axial top view is in the center of the circle of input points (412) and the stimulation field is displayed on an isosurface level which is identical to the level defined by the first input points (412).

15. The system (10) according to one of claims 6 to 14,
**characterized in that**
the adjustment means (400) comprises at least one touch screen (450), wherein the touch screen (450) is configured such that the at least one steering means (410) and the at least one visualization means (420) are provided by the touch screen (450).
